# FASCICULE DE BREVET EUROPEEN

(11) **EP 0 886 515 B1**
(45) Date de publication et mention de la délivrance du brevet: **16.01.2002**
(21) Numéro de dépôt: 97908306.0
(22) Date de dépôt: 05.03.1997
(51) Int. Cl.: A61K 7/06

(54) **UTILISATION DE N-ARYL-2-HYDROXYALKYLAMIDES POUR STIMULER OU INDUIRE LA POUSSE DES CHEVEUX ET/OU STOPPER LEUR CHUTE**
VERWENDUNG VON N-ARYL-2-HYDROXYALKYLAMIDEN ZUR INDUZIERUNG UND/ODER STIMULATION DES HAARWACHSTUMS UND/ODER ZUR VERLANGSAMUNG DES HAARAUSFALLS
USE OF N-ARYL-2-HYDROXYALKYLAMIDES FOR STIMULATING OR INDUCING HAIR GROWTH AND/OR ARRESTING HAIR LOSS

(30) Priorité: 06.03.1996 FR 9602828; 05.04.1996 FR 9604362
(43) Date de publication de la demande: 30.12.1998
(73) Titulaire: L'OREAL, 75008 Paris (FR)
(72) Inventeur: BRETON, Lionel, F-78000 Versailles (FR); GALEY, Jean-Baptiste, F-93600 Aulnay-sous-Bois (FR)
(74) Mandataire: Tezier Herman, Béatrice
(86) Numéro de dépôt international: FR9700389
(87) Numéro de publication internationale: WO9732562

(56) Documents cités:
- EP-A- 0 002 892
- EP-A- 0 040 932
- EP-A- 0 098 743
- EP-A- 0 524 781
- EP-A- 0 699 430
- DATABASE WPI Week 9008 Derwent Publications Ltd., London, GB; AN 90-056180 XP002017367 "Hair tonic cosmetic with good hair-growth-promoting action- containis odd-carbon fatty acids and aliphatic alcohol(s), and ortho-toluidine derivs." & JP 20 011 509 A (LION) , 16 Janvier 1990

## Description

La présente invention concerne l'utilisation à titre de principe actif, dans un milieu physiologiquement acceptable, dans une composition cosmétique ou pour la préparation d'une composition pharmaceutique, d'une quantité efficace d'au moins un composé particulier de la famille des N-aryl-2-hydroxyalkylamides, destinée à induire et/ou stimuler la croissance des cheveux et/ou freiner leur chute.

Chez l'être humain, la croissance des cheveux et leur renouvellement sont principalement déterminés par l'activité des follicules pileux. Leur activité est cyclique et comporte essentiellement trois phases, à savoir la phase anagène, la phase catagène et la phase télogène.

A la phase anagène active ou phase de croissance, qui dure plusieurs années et au cours de laquelle les cheveux s'allongent, succède une phase catagène très courte et transitoire qui dure quelques semaines, puis une phase de repos, appelée phase télogène, qui dure quelques mois.

A la fin de la période de repos, les cheveux tombent et un autre cycle recommence. La chevelure se renouvelle donc en permanence, et sur les 150 000 cheveux environ que comporte une chevelure, à chaque instant, 10% d'entre eux environ sont au repos et seront donc remplacés en quelques mois.

Cependant différentes causes peuvent entraîner une perte importante, temporaire ou définitive, des cheveux. L'alopécie est essentiellement due à une perturbation du renouvellement capillaire qui entraîne, dans un premier temps, l'accélération de la fréquence des cycles aux dépens de la qualité des cheveux puis de leur quantité. Il se produit un appauvrissement progressif de la chevelure par régression des cheveux dits "terminaux" au stade de duvets. Des zones sont touchées préférentiellement, notamment les golfes temporaux ou frontaux chez l'homme, et chez les femmes, on constate une alopécie diffuse du vertex.

Le terme alopécie recouvre toute une famille d'atteintes du follicule pileux ayant pour conséquence finale la perte définitive partielle ou générale des cheveux.
Dans un nombre important de cas, la chute précoce des cheveux survient chez des sujets prédisposés génétiquement et elle atteint notamment les hommes. Il s'agit plus particulièrement de l'alopécie androgénétique ou androgénique ou encore androgéno-génétique.

On recherche depuis de nombreuses années, dans l'industrie cosmétique ou pharmaceutique, des substances permettant de supprimer ou de réduire l'alopécie, et notamment d'induire ou de stimuler la croissance des cheveux ou de diminuer leur chute.

Dans cette optique, on a certes déjà proposé un grand nombre de composés actifs très divers, comme par exemple le 2,4-diamino 6-piperidinopyrimidine 3-oxyde ou "Minoxidil" décrit dans les brevets US 4 139 619 et US 4 596 812 ou encore ses nombreux dérivés comme ceux décrits par exemple dans les demandes de brevet EP 0353123, EP 0356271, EP 0408442, EP 0522964, EP 0420707, EP 0459890, EP 0519819.

Il reste, d'une manière générale, qu'il serait intéressant et utile de pouvoir disposer de composés actifs autres que ceux déjà connus, potentiellement plus actifs et/ou moins toxiques.

Ce but et d'autres sont atteints par la présente invention qui concerne l'utilisation à titre de principe actif pour induire et/ou stimuler la croissance des cheveux et/ou freiner leur chute, dans un milieu physiologiquement acceptable, dans une composition cosmétique, d'une quantité efficace d'au moins un composé répondant à la formule générale (I) : 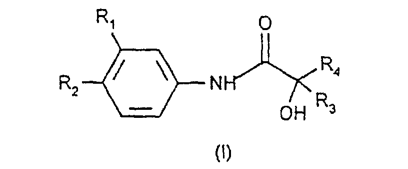 dans laquelle
R1 est un groupement cyano, un atome d'halogène ou un groupement alkyle, ayant de 1 à 4 atomes de carbone, substitué par au moins un atome d'halogène ;
R2 est un groupement cyano ou un atome d'halogène ;
R3 est un groupement alkyle, ayant 1 ou 2 atomes de carbone, éventuellement substitué par au moins un atome d'halogène ;
R4 est un atome d'hydrogène ou un groupement alkyle, ayant de 1 à 4 atomes de carbone, substitué par au moins un atome d'halogène ou un groupement aryle, éventuellement substitué par un ou plusieurs atomes d'halogène, par un ou plusieurs groupements hydroxy, carboxylique, nitro, cyano, alkyle linéaire ou ramifié, ayant 1 à 4 atomes de carbone, alcoxy linéaire ou ramifié, ayant 1 à 4 atomes de carbone, alcanoyle linéaire ou ramifié, ayant 1 à 4 atomes de carbone, perfluoroalkyle ;
ou d'au moins l'une de ses formes acylées ou encore d'au moins l'un de ses sels.

La présente invention concerne aussi l'utilisation, pour la préparation d'une composition pharmaceutique étant destinée à induire et/ou stimuler la croissance des cheveux et/ou freiner leur chute, d'une quantité efficace d'au moins un composé répondant à la formule générale (I), telle que définie ci-dessus, ou d'au moins une de ses formes acylées ou encore d'au moins un de ses sels.

Ces composés présentent des activités remarquables qui justifient leur utilisation comme principe actif pour induire et/ou stimuler la croissance des cheveux et/ou freiner leur chute.

Ils sont en effet d'excellents ouvreurs de canaux potassiques, propriété principale du Minoxidil, seul composé reconnu à ce jour comme efficace dans les traitements de la chute des cheveux.

Ils sont également d'excellents antagonistes réceptoriels des androgènes, les androgènes étant responsables d'une forme particulièrement répandue de l'alopécie, l'alopécie androgéno-dépendante.

A la connaissance de la demanderesse il n'a jamais été proposé dans l'art antérieur l'utilisation de tels composés à activité mixte, ouvreurs de canaux potassiques / anti-androgènes, pour lutter contre la chute des cheveux.

Selon une forme de réalisation particulière de l'invention, R1 peut être un atome de chlore, de brome ou de fluor, préférentiellement un atome de chlore.

Selon une autre forme de réalisation de l'invention, R1 peut être un groupement alkyle, ayant de 1 à 4 atomes de carbone, substitué par au moins un atome de chlore, de brome ou de fluor. Préférentiellement, R1 est un groupement alkyle, ayant de 1 à 4 atomes de carbone, substitué par au moins un atome de fluor, plus préférentiellement R1 est un radical alkyle perhalogéné, ayant de 1 à 4 atome de carbone, encore plus préférentiellement R1 est un radical méthyle perhalogéné et encore plus préférentiellement R1 est un radical méthyle perfluoré.

Lorsque R2 est un atome d'halogène, R2 peut être un atome de chlore, de brome
ou de fluor ; préférentiellement R2 est un atome de chlore.

R3 est de préférence un radical méthyle ou un radical méthyle perfluoré.

Lorsque R4 est un groupement alkyle ayant de 1 à 4 atomes de carbone substitué par au moins un atome d'halogène, le dit un atome d'halogène est choisi parmi le chlore, le brome ou le fluor, préférentiellement l'atome d'halogène est un atome de fluor.

Préférentiellement, R4 est un radical alkyle perhalogéné ayant de 1 à 4 atomes de carbone et encore plus préférentiellement R4 est un radical méthyle perhalogéné, préférentiellement un radical méthyle perfluoré.

Lorsque R4 est un groupement aryle, il est de préférence un radical phényle.

De préférence, R3 et R4 ont une signification différente.

Préférentiellement, le composé utilisé selon l'invention est choisi parmi le N-(4-cyano-3-trifluorométhyl-phényl)-3,3,3-trifluoro-2-hydroxy-2-méthylpropionamide, le N-(3-chloro-4-cyano-phényl)-3,3,3-trifluoro-2-hydroxy-2-méthylpropionamide, le N-(3,4-dichloro-phényl)-3,3,3-trifluoro-2-hydroxy-2-méthylpropionamide,le N-(4-cyano-3-trifluorométhyl-phényl)-2-hydroxy-2-phénylpropionamide et le N-(3,4-dicyano-phenyl)-3,3,3,-trifluoro-2-hydroxy-2-méthylpropionamide.

Ces composés peuvent être utilisés seuls ou en mélange.

La quantité efficace de composé à utiliser correspond bien entendu à la quantité nécessaire pour obtenir le résultat désiré. L'homme du métier est donc en mesure d'évaluer cette quantité efficace qui dépend de la nature du composé utilisé et de la personne ainsi traitée.
Pour donner un ordre de grandeur, selon l'invention, dans une composition cosmétique, le composé peut être présent à une concentration comprise entre 10⁻⁶ % et 5 %, en poids par rapport au poids total de la composition et de préférence comprise entre 10⁻³ % et 1 %. Dans la préparation d'une composition pharmaceutique, le composé peut être utilisé à une concentration comprise entre 10⁻⁵ % et 10 % en poids par rapport au poids total de la composition et de préférence comprise entre 10⁻² % et 2 %.

La composition pharmaceutique selon l'invention peut être administrée par voie parentérale, entérale ou encore par voie topique. De préférence, la composition pharmaceutique est administrée par voie topique.

Le milieu physiologiquement acceptable dans lequel l'actif est utilisé selon l'invention peut être anhydre ou aqueux. On entend par milieu anhydre, un milieu solvant contenant moins de 1% d'eau. Ce milieu peut être constitué d'un solvant
ou d'un mélange de solvants choisi plus particulièrement parmi les alcools inférieurs en C₂-C₄ comme l'alcool éthylique, les alkylèneglycols comme le propylèneglycol, et les alkyléthers d'alkylèneglycols ou de dialkylèneglycols, dont les radicaux alkyle ou alkylène contiennent de 1 à 4 atomes de carbone. On entend par milieu aqueux, un milieu constitué par de l'eau ou un mélange d'eau et d'un autre solvant physiologiquement acceptable, choisi notamment parmi les solvants organiques cités ci-dessus. Dans ce dernier cas, ces autres solvants, lorsqu'ils sont présents, représentent environ 5 à 95% en poids de la composition.

Il est possible que le milieu physiologiquement acceptable puisse contenir d'autres adjuvants habituellement utilisés dans le domaine cosmétique ou pharmaceutique, tels que des agents tensioactifs, des agents épaississants ou gélifiants, des agents cosmétiques, des agents conservateurs, des agents alcalinisants ou acidifiants bien connus dans l'état de la technique, et en quantités suffisantes pour obtenir la forme de présentation désirée, notamment de lotion plus ou moins épaissie, de gel, d'émulsion, ou de crème. L'utilisation peut éventuellement se faire sous une forme pressurisée en aérosol ou vaporisée à partir d'un flacon pompe.

Il est possible aussi d'utiliser en association avec l'actif des composés améliorant encore l'activité sur la repousse et/ou sur le freinage de la chute des cheveux, et ayant déjà été décrits pour cette activité.

Parmi ces derniers composés, on peut plus particulièrement citer à titre non limitatif :
- les esters d'acide nicotinique, dont notamment le nicotinate de tocophérol, le nicotinate de benzyle et les nicotinates d'alkyles en C₁-C₆ comme les nicotinates de méthyle ou d'hexyle ;
- les dérivés de pyrimidine, comme le 2,4-diamino 6-piperidinopyrimidine 3-oxyde ou "Minoxidil" décrit dans les brevets US 4 139 619 et US 4 596 812 ;
- les agents favorisant la repousse des cheveux comme ceux décrits par la demanderesse dans la demande de brevet européen publiée sous le numéro 0648488 ;
- les agents antibactériens tels que les macrolides, les pyranosides et les tétracyclines, et notamment l'érythromycine ;
- les agents antagonistes de calcium, comme la cinnarizine, le diltiazem, la nimodipine et la nifedipine ;
- des hormones, telles que l'estriol ou des analogues, ou la thyroxine et ses sels ;
- des agents anti-inflammatoires stéroïdiens, tels que les corticostéroïdes (par exemple : l'hydrocortisone) ;
- des agents antiandrogènes, tels que l'oxendolone, la spironolactone, le diéthylstilbestrol et la flutamide ;
- des inhibiteurs stéroïdiens ou non stéroïdiens des 5-α-réductases tels que le finastéride
- des agonistes potassiques tels que la cromakalim et le nicorandil.

A la liste ci-dessus, d'autres composés peuvent également être rajoutés, à savoir par exemple le diazoxyde, la spiroxasone, des phospholipides comme la lécithine, les acides linoléique et linolénique, l'acide salicylique et ses dérivés décrits dans le brevet français FR 2 581 542, comme les dérivés de l'acide salicylique porteurs d'un groupement alcanoyle ayant de 2 à 12 atomes de carbone en position 5 du cycle benzénique, des acides hydroxycarboxyliques ou cétocarboxyliques et leurs esters, des lactones et leurs sels correspondants, l'anthraline, des caroténoides, les acides eicosatétrayénoïque et eicosatriyénoïque ou leurs esters et amides, la vitamine D et ses dérivés, des extraits d'origine végétale ou bactérienne.

On peut également envisager que la composition comprenant au moins un composé tel que défini précédemment soit sous forme liposomée, telle que notamment décrite dans la demande de brevet WO 94/22468 déposée le 13 octobre 1994 par la société Anti Cancer Inc. Ainsi, le composé encapsulé dans les liposomes peut être délivré sélectivement au niveau du follicule pileux.

La composition cosmétique selon l'invention est à appliquer sur les zones alopéciques du cuir chevelu et des cheveux d'un individu, et est éventuellement laissée en contact plusieurs heures et est éventuellement à rincer. On peut, par exemple, appliquer la composition contenant une quantité efficace d'au moins un composé tel que défini précédemment, le soir, garder celle-ci au contact toute la nuit et éventuellement effectuer un shampooing le matin. Ces applications peuvent être renouvelées quotidiennement pendant un ou plusieurs mois suivant les individus.

Ainsi, la présente invention a également pour objet un procédé de traitement cosmétique des cheveux et/ou du cuir chevelu, caractérisé par le fait qu'il consiste à appliquer sur les cheveux et/ou le cuir chevelu, une composition comprenant une quantité efficace d'au moins composé tel que défini précédemment, à laisser celle-ci en contact avec les cheveux et/ou le cuir chevelu, et éventuellement à rincer.

Le procédé de traitement présente les caractéristiques d'un procédé cosmétique dans la mesure où il permet d'améliorer l'esthétique des cheveux en leur donnant une plus grande vigueur et un aspect amélioré.

On va maintenant donner à titre d'illustration des exemples qui ne sauraient limiter en aucune façon la portée de l'invention.

Exemple 1 : IC50 des composés déterminées pour leur effet sur les canaux potassiques et sur les récepteurs aux androgènes :

L'IC50 correspond à la concentration qui réduit de 50 % soit un mécanisme pharmacologique provoqué, comme la contraction musculaire, soit la fixation d'une molécule à son récepteur membranaire.

Pour déterminer l'IC50 des composés pour leur effet sur les canaux potassiques, ceux-ci on été testés selon les méthodes de Newgreen et col. (Br. J; Pharmacol., 1990, 100, 605-613), Bray et col. (Arch. Pharmacol., 1991, 344, 351-359), et Wickerden et col. (Br. J; Pharmacol., 1991, 103, 1148-1152):
Pour déterminer l'IC50 des composés pour leur effet sur les récepteurs aux androgènes, ceux-ci on été testés selon la méthode de Schilling et Liao ("The Prostate", 1984, 5, 581-588) :

Les valeurs sont données en µM.

Les composés suivants ont été testés :
A : N-(4-cyano-3-trifluorométhyl-phényl)-3,3,3-trifluoro-2-hydroxy-2-méthylpropionamide,
B : N-(3-chloro-4-cyano-phényl)-3,3,3-trifluoro-2-hydroxy-2-méthylpropionamide,
C : N-(3,4-dichloro-phényl)-3,3,3-trifluoro-2-hydroxy-2-méthylpropionamide,
D : N-(4-cyano-3-trifluorométhyl-phényl)-2-hydroxy-2-phénylpropionamide,
E : N-(3,4-dicyano-phenyl)-3,3,3,-trifluoro-2-hydroxy-2-méthylpropionamide,
F : N-(4-nitro-3-trifluorométhyl-phényl)- 3,3,3-trifluoro-2-hydroxy-2-méthylpropionamide ;
G : N-(4-cyano-phényl)-3,3,3-trifluoro-2-hydroxy-2-méthylpropionamide.
Les composés F et G de structures proches de celles des composés de l'invention, mais non inclus dans la définition des composés de l'invention, ont été testés à titre de comparatifs.

| Composé | Canaux K+ | Récepteurs aux androgènes |
|---|---|---|
| A | 0,5 | 1 |
| B | 0,1 | 0,5 |
| C | 2 | 1 |
| D | 3 | 3 |
| E | 1 | 10 |
| F | >10 | >10 |
| G | 10 | >10 |

Les molécules A, B, C, D et E présentent un bonne affinité pour le récepteur aux androgènes et un excellent effet ouvreur de canaux potassiques, alors qu'en comparaison les molécules F et G, proches de par leur structure mais n'entrant pas dans le cadre de l'invention, présentent une mauvaise affinité pour le récepteur aux androgènes et un mauvais effet ouvreur de canaux potassiques.

### Exemple 2 :

Exemples de compositions contenant un N-aryl-2-hydroxyalkylamide.

Ces compositions sont obtenues par les techniques habituelles couramment utilisées en cosmétique ou en pharmacie.

### Lotion antichute :

- N-(4-cyano-3-trifluorométhyl-phényl)-3,3,3-trifluoro-2-hydroxy-2-méthylpropionamide 0,1 g
- Propylène glycol 10,0 g
- Alcool isopropylique qsp 100,0 g

On applique 1 ml de cette lotion sur le cuir chevelu, à la fréquence de une à deux fois par jour.

### Gel niosomé :

| | |
|---|---|
| Chimexane NS① | 1,8 g |
| Stearoylglutamate monosodique | 0,2 g |
| N-(4-cyano-3-trifluorométhyl-phényl)-2-hydroxy-2-phénylpropionamide | 1,0 g |
| Carbomer | 0,2 g |
| Triéthanolamine qs | pH = 7 |
| Conservateurs qs | |
| Parfums qs | |
| Eau déminéralisée qsp | 100,0 g |

On applique ce gel sur le cuir chevelu, une à deux fois par jour.

### Lotion antichute :

- N-(3-chloro-4-cyano-phényl)-3,3,3-trifluoro-2-hydroxy-2-méthylpropionamide 0,5 g
- Propylène glycol 30,0 g
- Alcool éthylique 40,5 g
- Eau qsp 100,0 g

On applique cette lotion sur le cuir chevelu, une à deux fois par jour, à raison d' 1 ml par application.

### Lotion antichute épaissie :

- N-(4-cyano-3-trifluorométhyl-phényl)-3,3,3-trifluoro-2-hydroxy-2-méthylpropionamide 0,01 g
- Kawaïne 2,0 g
- Hydroxypropylcellulose vendue par la société Hercules sous la dénomination Klucel G 3,5 g
- Alcool éthylique qsp 100,0 g

On applique cette lotion épaissie sur le cuir chevelu, une à deux fois par jour, à raison d' 1 ml par application.

### Lotion niosomée :

| | |
|---|---|
| Chimexane NL① | 0,475 g |
| Cholestérol | 0,475 g |
| Stearoylglutamate monosodique | 0,05 g |
| N-(3,4-dicyano-phenyl)-3,3,3,-trifluoro-2-hydroxy-2-méthylpropionamide | 1,0 g |
| Conservateurs qs | |
| Colorants qs | |
| Parfum qs | |
| Eau déminéralisée qsp | 100,0 g |

On applique cette lotion sur le cuir chevelu, une à deux fois par jour, à raison d' 1 ml par application.

### lotion antichute :

- N-(3,4-dichloro-phényl)-3,3,3-trifluoro-2-hydroxy-2-méthylpropionamide 1,5 g
- Monométhyléther de propylèneglycol vendu sous la dénomination Dowanol PM par la société Dow Chemical 20,0 g
- Hydroxypropylcellulose vendue par la société Herculès sous la dénomination Klucel G 3,0 g
- Alcool éthylique 40,0 g
- Eau qsp 100,0 g

On applique cette lotion épaissie sur le cuir chevelu, une à deux fois par jour, à raison d' 1 ml par application.

Avec chacune des compositions décrites dans les exemples ci-dessus, on a constaté, après plusieurs mois de traitement et selon les sujets traités, un ralentissement de la chute des cheveux et/ou un effet repousse.

## Revendications

1. Utilisation cosmétique non thérapeutique, pour induire et/ou stimuler la croissance des cheveux et/ou freiner leur chute, dans une composition, d'une quantité efficace d'au moins un composé répondant à la formule générale (I) : 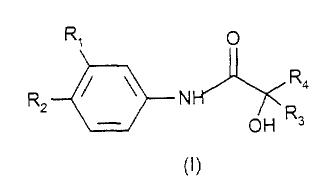 dans laquelle
R1 est un groupement cyano, un atome d'halogène ou un groupement alkyle, ayant de 1 à 4 atomes de carbone, substitué par au moins un atome d'halogène ;
R2 est un groupement cyano ou un atome d'halogène ;
R3 est un groupement alkyle, ayant 1 ou 2 atomes de carbone, éventuellement substitué par au moins un atome d'halogène ;
R4 est un atome d'hydrogène ou un groupement alkyle, ayant de 1 à 4 atomes de carbone substitué par au moins un atome d'halogène ou un groupement aryle, éventuellement substitué par un ou plusieurs atomes d'halogène, par un ou plusieurs groupements hydroxy, carboxylique, nitro, cyano, alkyle linéaire ou ramifié, ayant 1 à 4 atomes de carbone, alcoxy linéaire ou ramifié, ayant 1 à 4 atomes de carbone, alcanoyle linéaire ou ramifié, ayant 1 à 4 atomes de carbone, perfluoroalkyle ;
ou d'au moins l'une de ses formes acylées ou encore d'au moins l'un de ses sels.

2. Utilisation, pour la préparation d'une composition pharmaceutique, d'une quantité efficace d'au moins un composé répondant à la formule générale (I) : 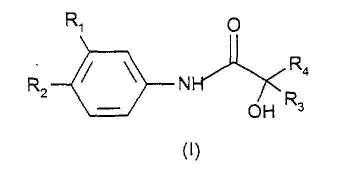 dans laquelle
R1 est un groupement cyano, un atome d'halogène ou un groupement alkyle, ayant de 1 à 4 atomes de carbone, substitué par au moins un atome d'halogène ;
R2 est un groupement cyano ou un atome d'halogène ;
R3 est un groupement alkyle, ayant 1 ou 2 atomes de carbone, éventuellement substitué par au moins un atome d'halogène ;
R4 est un atome d'hydrogène ou un groupement alkyle, ayant de 1 à 4 atomes de carbone substitué par au moins un atome d'halogène ou un groupement aryle, éventuellement substitué par un ou plusieurs atomes d'halogène, par un ou plusieurs groupements hydroxy, carboxylique, nitro, cyano, alkyle linéaire ou ramifié, ayant 1 à 4 atomes de carbone, alcoxy linéaire ou ramifié, ayant 1 à 4 atomes de carbone, alcanoyle linéaire ou ramifié, ayant 1 à 4 atomes de carbone, perfluoroalkyle ;
ou d'au moins l'une de ses formes acylées ou encore d'au moins l'un de ses sels; la composition pharmaceutique étant destinée à induire et/ou stimuler la croissance des cheveux et/ou freiner leur chute.

3. Utilisation selon l'une quelconque des revendications précédentes, **caractérisée par le fait que** R1 est un atome de chlore, de brome ou de fluor.

4. Utilisation selon la revendication précédente, **caractérisée par le fait que** R1 est un atome de chlore.

5. Utilisation selon l'une quelconque des revendications 1 et 2, **caractérisée par le fait que** R1 est un groupement alkyle ayant de 1 à 4 atomes de carbone substitué par au moins un atome de chlore, de brome ou de fluor.

6. Utilisation selon la revendication précédente, **caractérisée par le fait que** R1 est un groupement alkyle ayant de 1 à 4 atomes de carbone substitué par au moins un atome de fluor.

7. Utilisation selon la revendication précédente, **caractérisée par le fait que** R1 est un radical alkyle perhalogéné ayant de 1 à 4 atome de carbone.

8. Utilisation selon la revendication précédente, **caractérisée par le fait que** R1 est un radical méthyle perhalogéné.

9. Utilisation selon la revendication précédente, **caractérisée par le fait que** R1 est un radical méthyle perfluoré.

10. Utilisation selon l'une quelconque des revendications précédentes, **caractérisée par le fait que** R2 est un atome de chlore, de brome ou de fluor.

11. Utilisation selon la revendication précédente, **caractérisée par le fait que** R2 est un atome de chlore.

12. Utilisation selon l'une quelconque des revendications précédentes, **caractérisée par le fait que** R3 est un radical méthyle ou un radical méthyle perfluoré

13. Utilisation selon l'une quelconque des revendications précédentes, **caractérisée par le fait que** R4 est un groupement alkyle ayant de 1 à 4 atomes de carbone substitué par au moins un atome de chlore, de brome ou de fluor.

14. Utilisation selon la revendication précédente, **caractérisée par le fait que** R4 est un groupement alkyle ayant de 1 à 4 atomes de carbone substitué par au moins un atome de fluor.

15. Utilisation selon l'une des revendications 13 ou 14, **caractérisée par le fait que** le groupement alkyle substitué est un groupement alkyle perhalogéné.

16. Utilisation selon la revendication précédente, **caractérisée par le fait que** le groupement alkyle perhalogéné est un radical méthyle perhalogéné.

17. Utilisation selon l'une quelconque des revendications 1 à 12, **caractérisée par le fait que** R4 est un radical phényle.

18. Utilisation selon l'une quelconque des revendications précédentes, **caractérisée par le fait que** le composé est choisi parmi
le N-(4-cyano-3-trifluorométhyl-phényl)-3,3,3-trifluoro-2-hydroxy-2-méthylpropionamide,
le N-(3-chloro-4-cyano-phényl)-3,3,3-trifluoro-2-hydroxy-2-méthylpropionamide,
le N-(3,4-dichloro-phényl)-3,3,3-trifluoro-2-hydroxy-2-méthylpropionamide,
le N-(4-cyano-3-trifluorométhyl-phényl)-2-hydroxy-2-phénylpropionamide,
le N-(3,4-dicyano-phenyl)-3,3,3,-trifluoro-2-hydroxy-2-méthylpropionamide.

19. Utilisation dans une composition cosmétique selon l'une quelconque des revendications 1, 3 à 18, **caractérisée par le fait que** le composé présent est utilisé à une concentration comprise entre 10⁻⁶ % et 5 %, en poids par rapport au poids total de la composition et de préférence comprise entre 10⁻³ % et 1 %.

20. Utilisation pour la préparation d'une composition pharmaceutique selon l'une quelconque des revendications 2 à 18, **caractérisée par le fait que** le composé est utilisé à une concentration comprise entre 10⁻⁵ % et 10 % en poids par rapport au poids total de la composition et de préférence comprise entre 10⁻² % et 2 %.

21. Procédé de traitement cosmétique des cheveux et/ou du cuir chevelu, **caractérisé par le fait qu'**il consiste à appliquer sur les cheveux et/ou le cuir chevelu, une composition cosmétique telle que définie dans l'une quelconque des revendications 1 et 3 à 19, à laisser celle-ci en contact avec les cheveux et/ou le cuir chevelu, et éventuellement à rincer.

## Patentansprüche

1. Nicht therapeutische, kosmetische Verwendung mindestens einer Verbindung der allgemeinen Formel (I) in einer wirksamen. Menge in einer Zusammensetzung zur Induzierung und/oder Stimulation des Haarwachstums und/oder zur Verlangsamung des Haarausfalls : 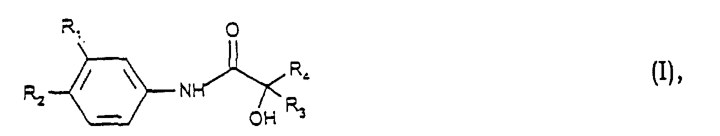 worin bedeuten:
R₁ eine Cyanogruppe, ein Halogenatom oder eine Alkylgruppe mit 1 bis 4 Kohlenstoffatomen, die mit mindestens einem Halogen substituiert ist;
R₂ eine Cyanogruppe oder ein Halogenatom;
R₃ eine Alkylgruppe mit 1 oder 2 Kohlenstoffatomen, die gegebenenfalls mit mindestens einem Halogenatom substituiert ist;
R₄ Wasserstoff, eine Alkylgruppe mit 1 bis 4 Kohlenstoffatomen, die mit mindestens einem Halogenatom substituiert ist, oder eine Arylgruppe, die gegebenenfalls mit einem oder mehreren Halogenatomen oder einer oder mehreren folgenden Gruppen substituiert ist: Hydroxy, Carboxy, Nitro, Cyano, geradkettiges oder verzweigtes Alkyl mit 1 bis 4 Kohlenstoffatomen, geradkettiges oder verzweigtes Alkoxy mit 1 bis 4 Kohlenstoffatomen, geradkettiges oder verzweigtes Alkanoyl mit 1 bis 4 Kohlenstoffatomen, Perfluoralkyl;
oder mindestens eine acylierte Form oder mindestens ein Salz dieser Verbindungen.

2. Verwendung mindestens einer Verbindung der allgemeinen Formel (I) in einer wirksamen Menge zur Herstellung einer pharmazeutischen Zusammensetzung: 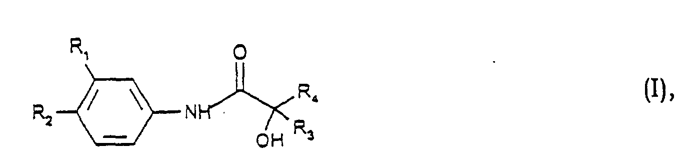 worin bedeuten:
R₁ eine Cyanogruppe, ein Halogenatom oder eine Alkylgruppe mit 1 bis 4 Kohlenstoffatomen, die mit mindestens einem Halogen substituiert ist;
R₂ eine Cyanogruppe oder ein Halogenatom;
R₃ eine Alkylgruppe mit 1 oder 2 Kohlenstoffatomen, die gegebenenfalls mit mindestens einem Halogenatom substituiert ist;
R₄ Wasserstoff, eine Alkylgruppe mit 1 bis 4 Kohlenstoffatomen, die mit mindestens einem Halogenatom substituiert ist, oder eine Arylgruppe, die gegebenenfalls mit einem oder mehreren Halogenatomen oder einer oder mehreren der folgenden Gruppen substituiert ist: Hydroxy, Carboxy, Nitro, Cyano, geradkettiges oder verzweigtes Alkyl mit 1 bis 4 Kohlenstoffatomen, geradkettiges oder verzweigtes Alkoxy mit 1 bis 4 Kohlenstoffatomen, geradkettiges oder verzweigtes Alkanoyl mit 1 bis 4 Kohlenstoffatomen, Perfluoralkyl;
oder mindestens eine acylierte Form oder mindestens ein Salz dieser Verbindungen, wobei die pharmazeutische Zusammensetzung zur Induzierung und/ oder Stimulation des Haarwachstums und/ oder zur Verlangsamung des Haarausfalls dienen soll.

3. Verwendung nach einem der vorhergehenden Ansprüche, **dadurch gekennzeichnet, daß** R₁ Chlor, Brom oder Fluor bedeutet.

4. Verwendung nach dem vorhergehenden Anspruch, **dadurch gekennzeichnet, daß** R₁ Chlor bedeutet.

5. Verwendung nach einem der Ansprüche 1 und 2, **dadurch gekennzeichnet, daß** R₁ eine Alkylgruppe mit 1 bis 4 Kohlenstoffatomen ist, die mit mindestens einem Chlor-, Brom- oder Fluoratom substituiert ist.

6. Verwendung nach dem vorhergehenden Anspruch, **dadurch gekennzeichnet, daß** R₁ eine Alkylgruppe mit 1 bis 4 Kohlenstoffatomen ist, die mit mindestens einem Fluoratom substituiert ist.

7. Verwendung nach dem vorhergehenden Anspruch, **dadurch gekennzeichnet, daß** R₁ ein perhalogeniertes Alkyl mit 1 bis 4 Kohlenstoffatomen ist.

8. Verwendung nach dem vorhergehenden Anspruch, **dadurch gekennzeichnet, daß** R₁ eine perhalogenierte Methylgruppe ist.

9. Verwendung nach dem vorhergehenden Anspruch, **dadurch gekennzeichnet, daß** R₁ die perfluorierte Methylgruppe ist.

10. Verwendung nach einem der vorhergehenden Ansprüche, **dadurch gekennzeichnet, daß** R₂ Chlor, Brom oder Fluor bedeutet.

11. Verwendung nach dem vorhergehenden Anspruch, **dadurch gekennzeichnet, daß** R₂ Chlor bedeutet.

12. Verwendung nach einem der vorhergehenden Ansprüche, **dadurch gekennzeichnet, daß** R₃ Methyl oder eine perfluorierte Methylgruppe bedeutet.

13. Verwendung nach einem der vorhergehenden Ansprüche, **dadurch gekennzeichnet, daß** R₄ eine Alkylgruppe mit 1 bis 4 Kohlenstoffatomen ist, die mit mindestens einern Chlor-, Brom- oder Fluoratom substituiert ist.

14. Verwendung nach dem vorhergehenden Anspruch, **dadurch gekennzeichnet, daß** R₄ eine Alkylgruppe mit 1 bis 4 Kohlenstoffatomen ist, die mit mindestens einem Fluoratom substituiert ist.

15. Venvendung nach einem der Ansprüche 13 oder 14, **dadurch gekennzeichnet, daß** die substituierte Alkylgruppe ein perhalogeniertes Alkyl ist.

16. Verwendung nach dem vorhergehenden Anspruch, **dadurch gekennzeichnet, daß** das perhalogenierte Alkyl eine perhalogenierte Methylgruppe ist.

17. Verwendung nach einem der Ansprüche 1 bis 12, **dadurch gekennzeichnet, daß** R₄ Phenyl bedeutet.

18. Verwendung nach einem der vorhergehenden Ansprüche, **dadurch gekennzeichnet, daß** die Verbindung ausgewählt ist unter:
- N-(4-Cyano-3-trifluormethyl-phenyl)-3,3,3-trifluor-2-hydroxy-2-methyl-propionamid;
- N-(3-Chlor-4-cyano-phenyl)-3,3,3-trifluor-2-hydroxy-2-methyl-propionamid;
- N-(3,4-Dichlor-phenyl)-3,3,3-trifluor-2-hydroxy-2-methyl-propionamid;
- N-(4-Cyano-3-trifluormethyl-phenyl)-2-hydroxy-2-phenyl-propionamid; und
- N-(3,4-Dicyano-phenyl)-3,3,3-trifluor-2-hydroxy-2-methyl-propionamid.

19. Verwendung in einer kosmetischen Zusammensetzung nach einem der Ansprüche 1, 3 bis 18, **dadurch gekennzeichnet, daß** die Verbindung in einer Konzentration von 10⁻⁶ bis 5 Gew.-%, bezogen auf das Gesamtgewicht der Zusammensetzung, und vorzugsweise 10⁻³ bis 1 % verwendet wird.

20. Verwendung zur Herstellung einer pharmazeutischen Zusammensetzung nach einem der Ansprüche 2 bis 18, **dadurch gekennzeichnet, daß** die Verbindung in einer Konzentration von 10⁻⁵ bis 10 Gew.-%, bezogen auf das Gesamtgewicht der Zusammensetzung, und vorzugsweise 10⁻² bis 2 % verwendet wird.

21. Verfahren zur kosmetischen Behandlung der Haare und/oder der Kopfhaut, **dadurch gekennzeichnet, daß** es darin besteht, daß auf die Haare und/ oder die Kopfhaut eine kosmetische Zusammensetzung nach einem der Ansprüche 1 und 3 bis 19 aufgetragen wird, diese mit den Haaren und/oder der Kopfhaut in Kontakt belassen wird und gegebenenfalls gespült wird.

## Claims

1. Non-therapeutic cosmetic use, for inducing and/or stimulating hair growth and/or slowing down hair loss, in a composition of an effective amount of at least one compound corresponding to the general formula (I) : 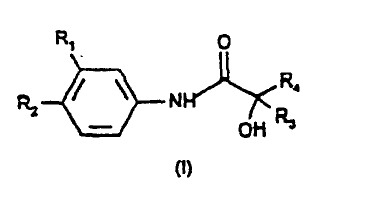 in which
R₁ is a cyano group, a halogen atom or an alkyl group, having from 1 to 4 carbon atoms, substituted by at least one halogen atom;
R₂ is a cyano group or a halogen atom;
R₃ is an alkyl group, having 1 or 2 carbon atoms, optionally substituted by at least one halogen atom;
R₄ is a hydrogen atom or an alkyl group, having from 1 to 4 carbon atoms, substituted by at least one halogen atom, or an aryl group, optionally substituted by one or more halogen atoms or by one or more hydroxyl, carboxyl, nitro, cyano, linear or branched alkyl, having 1 to 4 carbon atoms, linear or branched alkoxy, having 1 to 4 carbon atoms, linear or branched alkanoyl, having 1 to 4 carbon atoms, or perfluoroalkyl groups;
or of at least one of its acylated forms or alternatively of at least one of its salts.

2. Use, for the preparation of a pharmaceutical composition, of an effective amount of at least one compound corresponding to the general formula (I) : 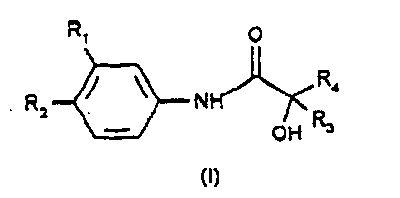 in which
R₁ is a cyano group, a halogen atom or an alkyl group, having from 1 to 4 carbon atoms, substituted by at least one halogen atom;
R₂ is a cyano group or a halogen atom;
R₃ is an alkyl group, having 1 or 2 carbon atoms, optionally substituted by at least one halogen atom;
R₄ is a hydrogen atom or an alkyl group, having from 1 to 4 carbon atoms, substituted by at least one halogen atom, or an aryl group, optionally substituted by one or more halogen atoms or by one or more hydroxyl, carboxyl, nitro, cyano, linear or branched alkyl, having 1 to 4 carbon atoms, linear or branched alkoxy, having 1 to 4 carbon atoms, linear or branched alkanoyl, having 1 to 4 carbon atoms, or perfluoroalkyl groups;
or of at least one of its acylated forms or alternatively of at least one of its salts; the pharmaceutical composition being intended to induce and/or stimulate hair growth and/or to slow down hair loss.

3. Use according to either one of the preceding claims, **characterized in that** R₁ is a chlorine, bromine or fluorine atom.

4. Use according to the preceding claim, **characterized in that** R₁ is a chlorine atom.

5. Use according to either one of Claims 1 and 2, **characterized in that** R₁ is an alkyl group, having from 1 to 4 carbon atoms, substituted by at least one chlorine, bromine or fluorine atom.

6. Use according to the preceding claim, **characterized in that** R₁ is an alkyl group, having from 1 to 4 carbon atoms, substituted by at least one fluorine atom.

7. Use according to the preceding claim, **characterized in that** R₁ is a perhalogenated alkyl radical having from 1 to 4 carbon atoms.

8. Use according to the preceding claim, **characterized in that** R₁ is a perhalogenated methyl radical.

9. Use according to the preceding claim, **characterized in that** R₁ is a perfluorinated methyl radical.

10. Use according to any one of the preceding claims, **characterized in that** R₂ is a chlorine, bromine or fluorine atom.

11. Use according to the preceding claim, **characterized in that** R₂ is a chlorine atom.

12. Use according to any one of the preceding claims, **characterized in that** R₃ is a methyl radical or a perfluorinated methyl radical.

13. Use according to any one of the preceding claims, **characterized in that** R₄ is an alkyl group, having from 1 to 4 carbon atoms, substituted by at least one chlorine, bromine or fluorine atom.

14. Use according to the preceding claim, **characterized in that** R₄ is an alkyl group, having from 1 to 4 carbon atoms, substituted by at least one fluorine atom.

15. Use according to either of Claims 13 and 14, **characterized in that** the substituted alkyl group is a perhalogenated alkyl group.

16. Use according to the preceding claim, **characterized in that** the perhalogenated alkyl group is a perhalogenated methyl radical.

17. Use according to any one of Claims 1 to 12, **characterized in that** R₄ is a phenyl radical.

18. Use according to any one of the preceding claims, **characterized in that** the compound is chosen from
N-(4-cyano-3-(trifluoromethyl)phenyl)-3,3,3-trifluoro-2-hydroxy-2-methylpropionamide,
N-(3-chloro-4-cyanophenyl)-3,3,3-trifluoro-2-hydroxy-2-methylpropionamide,
N-(3,4-dichlorophenyl)-3,3,3-trifluoro-2-hydroxy-2-methylpropionamide,
N-(4-cyano-3-(trifluoromethyl)phenyl)-2-hydroxy-2-phenylpropionamide,
N-(3,4-dicyanophenyl)-3,3,3-trifluoro-2-hydroxy-2-methylpropionamide.

19. Use in a cosmetic composition according to any one of Claims 1 and 3 to 18, **characterized in that** the compound present is used at a concentration of between 10⁻⁶% and 5%, by weight with respect to the total weight of the composition, and preferably of between 10⁻³% and 1%.

20. Use for the preparation of a pharmaceutical composition according to any one of Claims 2 to 18, **characterized in that** the compound is used at a concentration of between 10⁻⁵% and 10%, by weight with respect to the total weight of the composition; and preferably of between 10⁻²% and 2%.

21. Process for the cosmetic treatment of the hair and/or the scalp, **characterized in that** it consists in applying, to the hair and/or the scalp, a cosmetic composition as defined in any one of Claims 1 and 3 to 19, in leaving this composition in contact with the hair and/or the scalp, and optionally in rinsing.
